(19) Europäisches Patentamt
European Patent Office
Office européen des brevets

(11) Publication number: **0 009 702**
**B1**

(12) # EUROPEAN PATENT SPECIFICATION

(45) Date of publication of patent specification: **04.11.81**

(21) Application number: **79103473.9**

(22) Date of filing: **17.09.79**

(51) Int. Cl.³: **C 07 C 103/29,**
A 61 K 31/165 //C07C87/28

(54) **A phenylalkylaminoethylsalicylamide, its preparation and pharmaceutical compositions containing it.**

(30) Priority: **20.09.78 US 944516**

(43) Date of publication of application:
**16.04.80 Bulletin 80/8**

(45) Publication of the grant of the European patent:
**04.11.81 Bulletin 81/44**

(84) Designated Contracting States:
**AT BE CH DE FR GB IT NL SE**

(56) References cited:
**DE - A - 2 616 403**
**GB - A - 1 266 058**

(73) Proprietor: **SCHERING CORPORATION**
**2000 Galloping Hill Road**
**Kenilworth, New Jersey 07033 (US)**

(72) Inventor: **Gold, Elijah Herman**
**10 Roosevelt Avenue**
**West Orange Essex County New Jersey (US)**
Inventor: **Chang, Wei**
**63 West Cedar Street**
**Livingston Essex County New Jersey (US)**

(74) Representative: **Antony, Fritz, Dr. et al,**
**SCHERICO LTD. P.O. Box 601 Winkelriedstrasse 35**
**CH-6002 Lucerne (CH)**

**EP 0 009 702 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# A phenylalkylaminoethylsalicylamide, its preparation and pharmaceutical compositions containing it

This invention relates to a phenylalkylaminoethylsalicylamide, its preparation and pharmaceutical compositions containing it. More particularly it relates to one optical isomer of labetalol, 5 - [1 - hydroxy - 2 - (1 - methyl - 3 - phenylpropylamino) - ethyl]salicylamide.

The substance labetalol is known from British patent specification 1,266,058 and US—A—4,012,444. Its pharmacological properties are discussed by Farmer *et. al.* in *British Journal of Pharmacology,* 45: 660—675 (1972), who designate it AH5158; it is shown to block $\alpha$- and $\beta$-adrenergic receptors, suggesting that it would be useful in the treatment of arrhythmia, hypertension and angina pectoris.

The unique pharmacological properties of labetalol and its use as an antihypertensive agent are said to be largely a function of the exquisite balance of its $\alpha$- and $\beta$-blocking activities. The file history of US—A—4,012,444 indeed indicates that slight changes in the chemical structure of labetalol deleteriously affect this balance, and, even in the few analogous compounds where the balance is retained, the absolute potencies of these compounds are shown to be too low for them to be useful antihypertensive agents. Therefore, in the treatment of hypertension, labetalol is the compound of choice among those disclosed in British patent specification 1,266,058 and US—A—4,012,444.

Labetalol has two asymmetrically substituted carbon atoms and therefore can exist as two diastereoisomers and four optical isomers. Indeed, British patent specification 1,266,058 and US—A—4,012,444 disclose that compounds such as labetalol have optically active forms, but give no example of an optically active form. These patent specifications teach that "the racemic mixtures may be resolved by conventional methods, for example by salt formation with an optically active acid, followed by fractional crystallization", but give no method of resolution. Example 14 of each specification does indeed describe the separation of labetalol into two diastereoisomers "1" and "2", using benzoic acid, but this is not an optical resolution. In British patent specifications 1,541,932 and 1,541,933, "isomer 1" is designated "diastereoisomer A" and is characterised as that diastereoisomer whose hydrochloride salt has the higher melting point. These two British patent specifications also disclose that diastereoisomer A is a valuable antiarrhythmic agent since it has strongly reduced $\beta$-adrenergic blocking activity and is therefore useful in the treatment of people who have suffered myocardial infarction.

We have now discovered that diastereoisomer A is composed of the (S,R) and (R,S) optical isomers of labetalol, whereas diastereoisomer B is composed of the (S,S) and (R,R) optical isomers. We have also surprisingly found that the novel (R,R) optical isomer of labetalol exhibits, in comparison with labetalol itself, both an unexpectedly high increase in $\beta$-adrenergic blocking potency and a decrease in $\alpha$-adrenergig blocking potency. Thus, when the (R,R) optical isomer is compared with labetalol, the ratio of the $\beta$-adrenergic blocking potency to the $\alpha$-adrenergic blocking potency is found to be greatly and unexpectedly increased. In particular, animal tests have indicated that the (R,R) optical isomer has about twelve times the $\beta$-blocking potency of labetalol, but only about one third of the $\alpha$-blocking potency of labetalol. These properties could in no way have been predicted theoretically, especially as the $\beta$-blocking potency of diastereoisomer B is not significantly different from that of labetalol and the $\alpha$-blocking potency of diastereoisomer B is half that of labetalol. Indeed, it is clear, when the activities of the four optical isomers of labetalol are compare, that the activities of the diastereoisomers A and B and indeed of labetalol itself cannot be calculated from the activities of their components. One can put this the other way around by saying that the $\alpha$- and $\beta$-blocking activities of the four optical isomers of labetalol do not merely average to give the $\alpha$- and $\beta$-blocking activities of labetalol and of its diastereoisomers A and B. Some of the activities are much greater than could ever have been expected on a simple basis of mathematical proportions, in particular the high $\beta$-blocking activity of the (R,R) optical isomer: this activity is much higher than the $\beta$-blocking activity of diastereoisomer B so that antagonism evidently exists between the (S,S) and (R,R) optical isomers with respect to the $\beta$-blocking activity. This degree of antagonism could in no way have been foreseen. In the absence of this antagonism, the (R,R) optical isomer shows a balance of properties that make it the optical isomer of choice in the treatment of hypertension. In particular, the (R,R) optical isomer possesses potent antihypertensive activity and rapid onset of activity while substantially lacking the undesirable side-effects usually associated with $\alpha$-blockade, eg. postural hypotension.

The following Table shows the relationships between labetalol, its diastereoisomers A and B and the four pure optical isomers; below each compound are given its potencies as an $\alpha$-blocking and then as a $\beta$-blocking agent, all relative to the values for labetalol (assigned values 1.0 for each blocking activity):

labetalol

1.0; 1.0

| diastereoisomer A | | diastereoisomer B | |
|---|---|---|---|
| 1.2; 0.16 | | 0.5; 1.2 | |
| (S,R)-isomer | (R,S)-isomer | (S,S)-isomer | (R,R)-isomer |
| 4.9; 0.05 | 0.4; 0.3 | 0.8; 0.02 | 0.3; 12.3 |

This table clearly shows the unexpectedly high $\beta$-blocking activity and ratio of $\beta$-:$\alpha$-blocking activities possessed by the (R,R)-optical isomer. Additionally, the (R,R)-optical isomer has been found to possess greater direct peripheral vasodilation activity than labetalol, and this also contributes to its anti-hypertensive activity. Moreover, the (R,R)-optical isomer is substantially non-toxic at therapeutic doses.

According to the invention therefore we provide the (R,R)-optical isomer of labetalol, namely 5 - {(R) - 1 - hydroxy - 2 - [(R) -(1 - methyl - 3 - phenylpropyl)amino]ethyl}salicylamide, which can be characterised by means of its hydrochloride salt which is dimorphic with m.pts. of about 133—134°C. and about 192—193.5°C. and an $[\alpha]_D^{26}$ of about −30.6° (conc. 1 mg./ml., ethanol), said (R,R) optical isomer being substantially free of the corresponding (R,S), (S,R) and (S,S) optical isomers, and the pharmaceutically acceptable salts thereof. Such salts include especially the above-mentioned hydrochloride salt, the sulfate, maleate, tartrate and citrate salts, and also the acetate, phthalate, succinate, lactate, maleate, cinnamate, hydrobromide and phosphate salts.

As far as we know, no one has succeeded in resolving labetalol by standard methods and no individual optical isomer of labetalol has yet been disclosed. Indeed, we ourselves have tried several standard methods of resolving labetalol by salt formation with an optically active acid, but all these standard methods have failed. In particular, all out attempts to resolve diastereoisomer B of labetalol using the following reagents have failed: N-acetyl-L-leucine, N-tosyl-L-(+)-glutamic acid, N-tosyl-L-leucine and N-tosyl-D-leucine. We have therefore concluded that attempts to resolve diastereoisomer B by such methods are likely to fail and that stereospecific syntheses most probably offers a more fruitful route to the individual isomers of labetalol and in particular to the (R,R) optical isomer thereof. An especially preferred embodiment of such a stereospecific syntheses is shown in the following reaction scheme:

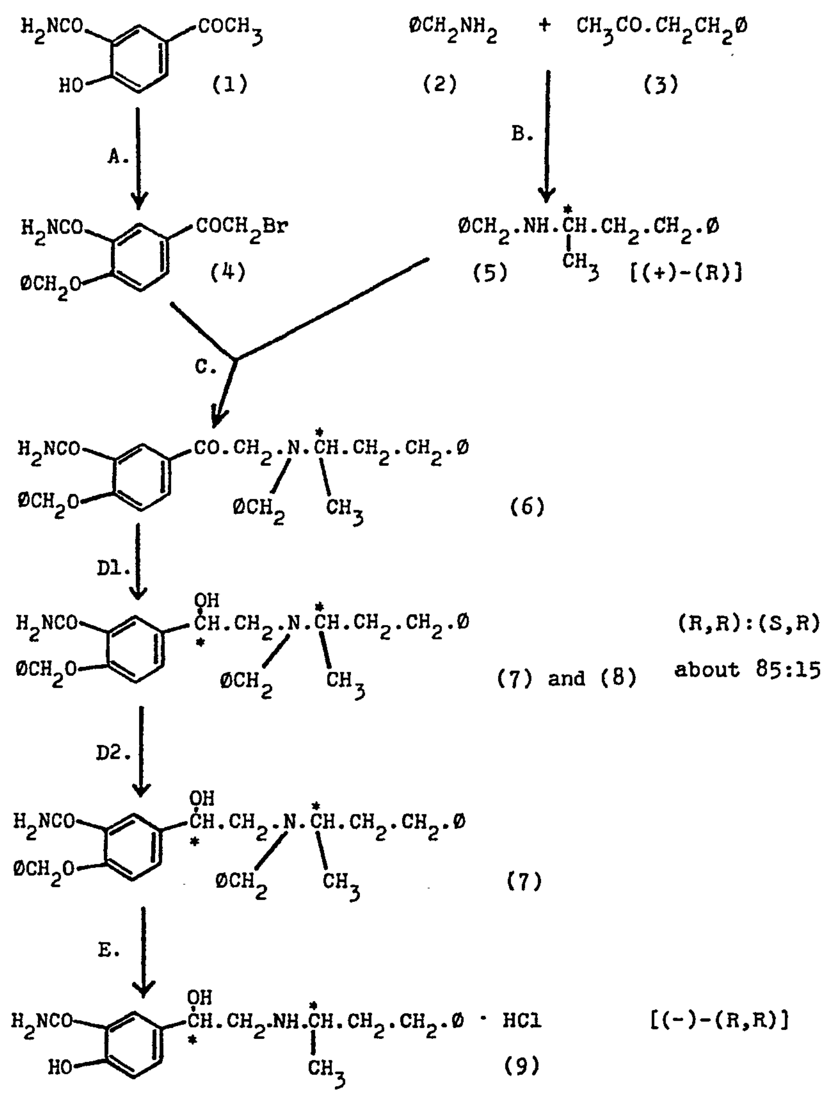


In the reaction scheme:

compounds (6), (7), (8) and (9) are novel and are features of the invention; "Ø" shows an unsubstituted phenyl group and asterisks indicate asymmetrically substituted carbon atoms; and the various steps can be described in general terms as follows:

A: (i) Formation of phenolate salt with a strong base in an organic solvent;

(ii) introduction of a protecting group on to the phenolic oxygen atom;

(iii) bromination with bromine in an inert organic solvent.

B: (i) Condensation to a Schiff's base, e.g. by heating in a water-immiscible organic solvent in the presence of an acid catalyst with azeotropic removal of water;

(ii) reduction of the Schiff's base to a secondary amine;

(iii) resolution of this secondary amine into its optical antipodes and isolation of the (+)-(R)-optical isomer.

C: Condensation to a tertiary amine by reaction in an inert organic solvent in the presence of an acid binding agent.

D1: Reduction

D2: Separation of isomers, e.g. by chromatography.

E: Hydrogenolysis of protecting groups with hydrogen and a catalyst and isolation of the (R,R) optical isomer e.g. as an acid additon salt.

The novel and inventive process is of course not restricted exactly to the sequence of steps given above.

According to the invention, one process for the preparation of the novel (R,R) optical isomer of labetalol and of its pharmaceutically acceptable acid addition salts, substantially free of the corresponding (R,S), (S,R) and (S,S) optical isomers, comprises removing the protecting groups from an N,O-protected (R,R) optical isomer or acid addition salt thereof, the term "N,O-protected" indicating that the basic nitrogen atom and the phenolic hydroxy group are protected, and isolating the resulting (R,R) optical isomer as the free base or as a pharmaceutically acceptable acid addition salt.

The protecting groups are preferably such groups as can be removed by hydrogenolysis, e.g. by means of hydrogen and palladium on carbon; examples of such groups include the N- or O-benzyl, N- or O-benzyloxycarbonyl, N- or O-benzhydryl, N-trichloroethoxycarbonyl or N-trityl group. Preferably both protecting groups are benzyl groups. Alternatively, the protecting groups can be such groups as are removable by mild hydrolysis, e.g., an N- or O-benzyloxycarbonyl or N-trifluoroacetyl group.

The N,O-protected (R,R) optical isomer used as starting material in the above process according to the invention is preferably obtained by resolution of an N,O-protected (R,R) (S,R) diastereoisomeric mixture. This resolution can conveniently be effected by physical methods, e.g. by chromatography especially on silica gel. This N,O-protected (R,R) (S,R) diastereoisomeric mixture can be obtained by reduction of an N,O-protected 5 - {N - [(R) - 1 - methyl - 3 - phenylpropyl]glycyl}salicylamide, preferably by means of a borohydride in an organic solvent, especially sodium borohydride in a lower alkanol such as methanol or ethanol. Other reducing agents that can conveniently be used include lithium borohydride or an alkali metal alkylborohydride, e.g. lithium or potassium tri-*s*-butylborohydride or lithium or potassium tri-(3-methyl-2-butyl)borohydride, in an organic solvent such as tetrahydrofuran.

The N,O-protected 5 - {N - [(R) - 1 - methyl - 3 - phenylpropyl]glycyl}salicylamide can be obtained by condensation of a 4 - O - protected - $\alpha$ - bromo - 3 - carbamoylacetophenone with an (R) - N - protected - 1 - methyl - 3 - phenylpropylamine, for example in the presence of an excess of the latter reagent, or of triethylamine or 2,2,6,6-tetramethylpiperidine or especially of potassium carbonate as acid binding agent, and also of dimethylformamide as organic solvent.

The 4 - O - protected - $\alpha$ - bromo - 3 - carbamoylacetophenone can be prepared by protection of the phenolic hydroxy group of 5-acetylsalicylamide, e.g. by formation of an alkali salt and benzylation with benzylchloride; the bromine atom in the side chain can then be introduced by bromination with bromine in chloroform.

The (R) - N - protected - 1 - methyl - 3 - phenylpropylamine can be prepared by condensation of an amine carrying a suitable protecting group, e.g. benzylamine, with benzyl acetone, preferably under reflux in a water-immiscible organic solvent and in the presence of a strong acid catalyst e.g. *p*-toluenesulfonic acid, with continuous removal of water. The resulting Schiff's base is then reduced under mild conditions, e.g. with sodium borohydride in methanol, so as not to remove the protecting group. Alternatively, the N-protecting group can be directly introduced into 1-methyl-3-phenylpropylamine. The N-protected 1-methyl-3-phenylpropylamine can then be resolved and the (+)-(R)-optical isomer isolated, e.g. with N-*p*-toluenesulfonyl-(L)-leucine followed by N-acetyl-(L)-leucine, or with N-*p*-toluenesulfonyl-(D)-leucine. Other reagents that may be used include 2,3,5,6-di-O-isopropylidene-2-keto-(L)-gulonic acid and the D- and L- forms of dibenzoyl tartaric acid, ditoluoyl tartaric acid and mandelic acid. If desired, the resolution can be deferred until step C (condensation) has been carried out.

A further feature of the present invention comprises a process for the preparation of the (R,R) optical isomer of labetalol and its pharmaceutically acceptable acid addition salts, substantially free of the corresponding (R,S), (S,R) and (S,S) optical isomers, consisting of the steps of condensing a 4-O-protected-$\alpha$-bromo-3-carbamoylacetophenone with an N-protected-1-methyl-3-phenylpropylamine, obtaining an N,O-protected 5 - {N - [(R) - 1 - methyl - 3 - phenylpropyl]glycyl}salicylamide either by using an (R) - N - protected - 1 - methyl - 3 - phenylpropylamine in the foregoing condensation or by resolving the resulting racemic N,O-protected 5 - [N - (1 - methyl - 3 - phenylpropyl)glycyl]salicylamide, reducing the N,O-protected 5 - {N - [(R) - 1 - methyl - 3 - phenylpropyl]glycyl}salicylamide to a mixture of N,O-protected 5 - {(R) - 1 - hydroxy - 2 - [(R) - (1 - methyl - 3 - phenylpropyl)amino]ethyl}salicylamide and the corresponding (S,R) optical isomer, separating from this mixture the N,O-protected 5 - {(R) - 1 - hydroxy - 2 - [(R) - (1 - methyl - 3 - phenylpropyl)amino]ethyl}salicylamide, removing the protecting groups therefrom and isolating 5 - {(R) - 1 - hydroxy - 2 - [(R) - (1 - methyl - 3 - phenylpropyl)amino]ethyl}salicylamide as the free base or as a pharmaceutically acceptable acid addition salt thereof.

A preferred feature of this process comprises the steps of condensing 4-benzyloxy-$\alpha$-bromo-3-carbamoylacetophenone with (R) - (+) - N - benzyl - 1 - methyl - 3 - phenylpropylamine to yield 2 - O - benzyl - 5 - {N - benzyl - N - [(R) - 1 - methyl - 3 - phenylpropyl]glycyl}salicylamide, reducing this compound to a mixture of 2 - O - benzyl - 5 - {(R) - 1 - hydroxy - 2 - [(R) - (1 -

methyl - 3 - phenylpropyl)benzylamino]ethyl}salicylamide and the corresponding (S,R) optical isomer, separating from this mixture 2 - O - benzyl - 5 - {(R) - 1 - hydroxy - 2 - [(R) - (1 - methyl - 3 - phenylpropyl)benzylamino]ethyl}salicylamide, removing the protecting N- and O-benzyl groups therefrom by hydrogenolysis and isolating 5 - {(R) - 1 - hydroxy - 2 - [(R) - 1 - methyl - 3 - phenylpropyl)amino]ethyl}salicylamide as the free base or as a pharmaceutically acceptable acid addition salt thereof.

The preferred process can be carried out under conditions described above. Thus the step of condensing is preferably effected in the presence of potassium carbonate as acid binding agent and of dimethylformamide as inert organic solvent; the step of reducing is preferably effected by means of an alkali metal borohydride in an inert organic solvent; and the step of separating is conveniently effected by chromatography.

The R,R-isomer can be isolated as the free base or as a pharmaceutically acceptable acid addition salt. These salts can be prepared by standard methods.

The hydrochloride salt of the novel (R,R)-optical isomer is dimorphic and exists in two crystalline forms m.pt. about 133—134°C. and about 192—193.5°C. The characterising constants given in the Example (part E) are for the higher melting (presumably the thermodynamically more stable) crystalline form, although either form may be obtained.

The α- and β-blocking activities discussed earlier in this specification can be determined by known methods, e.g. by those of Farmer *et. al., Brit. J. Pharm., 45,* 660 (1972); Robson, *J. Pharm. Exp. Therap., 175,* 157 (1970), and Levy, *Arch. Int. Pharmacodyn. Ther., 204,* 143 (1973).

The following Example describes the preparation of the active compound of the present invention and intermediates in its preparation.

EXAMPLE

A. *4-Benzyloxy-α-bromo-3-carbamoylacetophenone (4)*

To a solution of 1115.4 g. (0.644 mol.) of 5-acetylsalicylamide (1) in 1.2 liter of dimethylformamide add 33.1 g. (0.613 mol.) of sodium methoxide in small portions with cooling and stirring. Heat the mixture on a steam bath and add 75 ml. (0.652 mol.) of benzylchloride dropwise. Continue heating and stirring for 7 hours. After stirring and cooling, pour the mixture into 6 liters of ice-water containing 15 g. of sodium carbonate. Filter, wash well with water, digest with 700 ml. of ethanol, chill and refilter to obtain analytically pure 4 - benzyloxy - 3 - carbamoylacetophenone, m.p. 157—160°C.

To a refluxing, stirred solution of 127.0 g. (0.47 mol.) of 4 - benzyloxy - 3 - carbamoylacetophenone in 1.2 liter of chloroform, add a few ml. of a solution of 76.5 g. (0.49 mol.) bromine in 220 ml. of chloroform and wait until the colour is discharged (ca. 5—10 min.). Cool the hot solution to room temperature and add the remaining bromine solution dropwise with stirring at room temperature until precipitation begins; then reflux the reaction mixture and continue the dropwise addition. After refluxing for 10 min. following completion of the addition, chill the solution in an ice bath, filter off the solid and wash it with cold chloroform. Stir the crude solid for 20 min. in 800 ml. of ice-cold water, filter it off, wash it well with water and dry it. Recrystallize it from methylethyl ketone to afford two crops of the product (4), m.p. 150—152°C. and m.p. 146—149°C., both of which are usable for the preparation of 2 - O - benzyl - 5 - {N - benzyl - N - [(R) - 1 - methyl - 3 - phenylpropyl]glycyl}salicylamide (6).

B. *(R)-(+)-N-Benzyl-1-methyl-3-phenylpropylamine (5)*

In an apparatus fitted with a Dean and Stark trap, reflux a solution of 1.0 kg. (6.75 mol.) of benzylacetone (3), 725 g. (6.75 mol.) of benzylamine (2) and 5.0 g. of *p*-toluenesulfonic acid hydrate in 7 liters of benzene for 14 hours. Remove the solvent *in vacuo* and dissolve the residue in 6.5 liters of methanol. With cooling and stirring, carefully add 125 g. of sodium borohydride and stir the mixture for 16 hours at room temperature. Remove the methanol *in vacuo*, add 2 liters of water and 4 liters of benzene, and extract the product into the benzene. Dry the solution over anhydrous magnesium sulfate, filter, and distil the filtrate, collecting the fraction b.p. 145—150°C./0.5 mm. Dissolve 1,028 g. (4.288 mol.) of the distillate and 1,230 g. (4.328 mol.) of N-*p*-toluenesulfonyl-(L)-leucine in 7.2 liters of boiling ethanol and allow to cool to room temperature without agitation. Wash the resulting precipitate with a small amount of ice-cold ethanol, recrystallize it from 4.8 liters of ethanol, filter off the solid product and wash it with ice-cold ethanol. This solid product is highly enriched with the salt of the undesired (S)-enantiomer. Combine the mother liquors from the original precipitation and from the recrystallization, remove the solvent and recover the free base by basifying with 500 ml of 20% aqueous sodium hydroxide and extracting with benzene. After drying over anhydrous magnesium sulfate, filtering and removing the benzene, dissolve the residue [487 g. (2.04 mol.)] and 346 g. (2.06 mol.) of N-acetyl-(L)-leucine in 2.0 liters of boiling ethanol and allow the solution to cool to room temperature. Filter off the product and recrystallize it once from 1.8 liter of ethanol and then from 4.0 liters of acetonitrile to obtain the salt of the desired (R)-enantiomer with N-acetyl-(L)-leucine, m.p. 151—152°C. Basify with 400 ml. of aqueous 2.5N sodium hydroxide, extract with ether, dry over anhydrous magnesium sulfate, filter and remove the solvent *in vacuo* to obtain the product (5), $[\alpha]_D^{26} = +4.5°$ (c = 5.0, ethanol).

C. *2 - O - Benzyl - 5 - {N - benzyl - N - [(R) - 1 - methyl - 3 - phenylpropyl]glycyl}salicylamide (6)*

Stir a mixture of 224 g. (0.94 mol.) of (R) - (+) - N - benzyl - 1 - methyl - 3 - phenylpropylamine (5), 372 g. (*ca.* 1.07 mol.) of 4 - benzyloxy - $\alpha$ - bromo - 3 - carbamoylacetophenone (4) and 372 g. (2.7 mol.) of potassium carbonate in 1.6 liters dimethylformamide at room temperature for 4 hours (reaction mildly exothermic). Add 8.7 liters water and extract with ether, dry over anhydrous sodium sulfate, filter, and remove the ether *in vacuo* (up to 30—40°C.) to yield the crude product (6) as a syrup.

D. *2 - O - Benzyl - 5 - {(R) - 1 - hydroxy - 2 - [(R) - (1 - methyl - 3 - phenylpropyl) benzylamino]ethyl}salicylamide (7)*

1.(a) Dissolve 520 g. (not more than 0.94 mol.) of crude 2 - O - benzyl - 5 - {N - benzyl - N - [(R) - 1 - methyl - 3 - phenylpropyl]glycyl}salicylamide (6) in 3.1 liters ethanol and, with stirring and cooling, add portionwise 35.5 g. (0.94 mol.) of sodium borohydride. Stir the mixture at room temperature for 16 hours, remove the solvent *in vacuo*, add 3.2 liters water and heat the mixture for 30 minutes on a steam bath. Cool, extract with benzene, dry the benzene layer over anhydrous magnesium sulfate, filter, remove the solvent *in vacuo*, and obtain the crude product as a syrup (ratio R,R:S,R *ca.* 85:15).

The following procedures show that this reduction can be carried out by a variety of further reducing agents:

(b) Add one ml. of 0.5M potassium tri-*sec*-butylborohydride solution in tetrahydrofuran to a cold solution of 200 mg. of the crude aminoketone (6) from step C in 10 ml. of tetrahydrofuran with stirring and cooling in an ice bath. Stir for another thirty minutes. Heat a sample with a few drops of methanol; thin layer chromatography on silica gel using chloroform:ethyl acetate (3:1) as developing solvent shows that the ratio of isomers (R,R):(S,R) in the product is about 70:30.

(c) Add 0.5 ml. of 1.0M lithium tri-*sec*-butylborohydride solution in tetrahydrofuran to a cold solution of 100 mg. of the crude aminoketone (6) from step C in 10 ml. of tetrahydrofuran with stirring and cooling in an ice bath. Stir for another ten minutes, then hydrolyse with a few drops of ethanol. Distil off the solvent and extract the residue with benzene and water. Separate the benzene layer, dry it over anhydrous sodium sulfate, filter and distil off the benzene. The ratio of isomers (R,R):(S,R) in the product is shown by pmr spectrum to be about 80:20.

(d) Replacement of the solvent (10 ml.) of tetrahydrofuran) for the crude aminoketone (6) with 10 ml. of benzene in procedures (b) and (c) gives almost identical results to those of procedures (b) and (c).

(e) Dissolve 63.0 g. of crude aminoketone (6) from step C in a mixture of 960 ml. of benzene and 40 ml. of tetrahydrofuran, and decant off the solution from insoluble material. Add this solution dropwise to a suspension of 4.20 g. of lithium borohydride in 480 ml. of benzene and 20 ml. of tetrahydrofuran with stirring and cooling in an ice-bath. Stir for an additional two hours, then decompose with water. Separate the organic layer and dry it over anhydrous sodium sulfate. Filter and evaporate the filtrate to dryness on a rotary evaporator. The ratio of isomers (R,R):(S,R) in the product is shown by pmr spectrum to be about 70:30.

2.(a) Chromatograph 47 g. of the crude mixture from e.g. 1.(a) above on 1.5 kg. of thin layer grade silica gel with chloroform:ethyl acetate (3:1) and obtain the pure product (7), which is eluted first. This compound's pmr spectrum in $CDCl_3$ is assigned as follows: $\delta = 1.02$ (d,C—$CH_3$; J = 7 Hz), 1.42—2.00 (m,—$C(CH_3)$—$CH_2$—), 3.66 (q,N—$CH_2$—$C_6H_5$), 4.62 (q,$C$HOH), 5.15 (s,O$CH_2$—$C_6H_5$).

(b) The product from 1(b) to (e) can similarly be resolved: e.g., chromatography of the product (53 g.) from 1(e) on 1,200 g. of a silica gel column using chloroform:ethyl-acetate (3:1) provides the pure product (7).

E. *(—) - 5 - {(R) - 1 - Hydroxy - 2 - [(R) - (1 - methyl - 3 - phenylpropyl)amino]ethyl}salicylamide hydrochloride salt (9)*

Treat a solution of 3.0 g. (0.0059 mol.) of 2 - O - benzyl - 5 - {(R) - 1 - hydroxy - 2 - [(R) - (1 - methyl - 3 - phenylpropyl)benzylamino]ethyl}salicylamide in 30 ml. of ethyl ether with 2N ethereal hydrogen chloride until no further precipitation occurs. Wash the precipitated 2 - O - benzyl - 5 - {(R) - 1 - hydroxy - 2 - [(R) - (1 - methyl - 3 - phenylpropyl)benzylamino]ethyl}salicylamide hydrochloride with ether to remove excess hydrogen chloride and dissolve it in 100 ml. ethanol. To the ethanol solution add 300 mg. of a 20% palladium hydroxide on carbon catalyst and hydrogenate (3 atm.; 3.1 kg. $cm^{-2}$) in a Paar apparatus with shaking at room temperature for 3 hours. Filter off the catalyst, evaporate, and triturate the solid residue with isopropanol. Dissolve the solid in 11 ml. of 1N sodium hydroxide, adjust the pH to about 8 and precipitate the free base by bubbling in carbon dioxide. Collect the free base, wash it with water and dry it in vacuo at 40°C. Chromatograph the free base on 450 g. of silica gel and dissolve the pure product in 20 ml. of boiling acetonitrile. Cool the solution and carefully acidify with 2N ethereal HCl to about pH2. Solidify the gum which precipitates by refluxing the mixture for 10 minutes, filter off the solid, wash it with ethyl ether and recrystallize it from ethanol to obtain analytically pure product (9), m.p. 192—193.5°C. (dec.), $[\alpha]_D^{26} = -30.6°$ (c = 1.0, ethanol).

The (R,R) isomer and its pharmaceutically acceptable salts are useful in the treatment of

cardiovascular disorders and particularly in the treatment of mammalian hypertension. They can also be used for direct peripheral vasodilation and in the treatment of glaucoma. They are preferably administered orally but can also be administered by injection. Laboratory tests indicate that the effective oral dose ($ED_{50}$) for the (R,R) isomer or a pharmaceutically acceptable salt thereof will typically lie within the range of 0.01 to 25 mg./kg., preferably 0.5 to 5 mg./kg., of mammalian weight.

The invention therefore provides pharmaceutical compositions containing as active ingredient 5 - {(R) - 1 - hydroxy - 2 - [(R) - (1 - methyl - 3 - phenylpropyl)amino]ethyl}salicylamide or a pharmaceutically acceptable acid addition salt thereof, together with a pharmaceutical carrier or excipient, said (R,R) optical isomer being substantially free of the corresponding (R,S), (S,R) and (S,S) optical isomers. The compositions are preferably in the form of dosage units, e.g. tablets, pills, capsules, suppositories or injectable preparations in ampoules. The compositions may also be for example in the form of powders, syrups, elixirs or suspensions. The required daily dosage may be administered in single or divided doses. The exact dose to be administered will, of course, be dependent upon various factors such as the age and weight of the subject mammal and the individual response. Dosage units preferably contain from 2 to 500 mg., more preferably from 10 to 200 mg., of the (R,R) isomer according to the invention (or pharmaceutically acceptable acid addition salt thereof).

Typical pharmaceutically acceptable carriers for use in formulations described above are exemplified by: sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tri-calcium phosphate; sodium sulfate; calcium sulfate; polyvinylpyrrolidone; polyvinyl alcohol; stearic acid; alkaline earth metal stearates such as magnesium stearate and calcium stearate; vegetable oils such as peanut oil, cottonseed oil, sesame oil, olive oil and corn oil; non-ionic, cationic and anionic surfactants; ethylene glycol polymers; $\beta$-cyclodextrin; fatty alcohols; hydrolyzed cereal solids; and other non-toxic compatible fillers, binders, disintegrants, and lubricants commonly used in pharmaceutical formulations.

In treating certain patients with the R,R-isomer of this invention, it may be desirable to include other pharmaceutically active ingredients in the same composition. For example, in treating patients in whom salt and water retention is a problem, effective amounts of a diuretic, *e.g.,* hydrochlorothiazide or trichloromethiazide, may be included.

*Pharmaceutical Formulations*

In the following examples, the active ingredient is preferably 5 - {(R) - 1 - hydroxy - 2 - [(R) - (1 - methyl - 3 - phenylpropyl)amino]ethyl}salicylamide hydrochloride, but an equivalent quantity of the (R,R) isomer itself or of another pharmaceutically acceptable acid addition salt, especially a salt named herein, may be substituted:

| Injectable Solution | mg./ml. |
|---|---|
| Active ingredient | 5.00 |
| Methyl *p*-hydroxybenzoate | 0.80 |
| Propyl *p*-hydroxybenzoate | 0.10 |
| Disodium Edetate | 0.10 |
| Citric Acid Monohydrate | 0.08 |
| Dextrose | 40.00 |
| Water for injection qs ad | 1.00 ml. |

*Manufacturing Procedure:*

Dissolve the *p*-hydroxybenzoates in a portion of water for injection at 60—70°C., and cool the solution to 25—35°C. Charge and dissolve all other excipients and the active ingredient. Bring the solution to final volume, filter it through a sterilizing membrane and fill into sterile containers.

*Oral Formulations:*

*a) Capsules:*

| Formula | Quantities per capsule (mg.) | (mg.) |
|---|---|---|
| Active ingredient | 200.0 | 100.0 |
| Lactose | 223.0 | 111.5 |
| Corn Starch | 75.0 | 37.5 |
| Magnesium Stearate | 2.0 | 1.0 |
| | 500.0 | 250.0 |

*Manufacturing Procedure:*

Blend the active ingredient, lactose and corn starch until uniform; then blend the magnesium stearate into the resulting powder. Encapsulate the mixture into suitably sized two-piece hard gelatin capsules.

b) *Tablets*

| Formula | Quantities per tablet (mg.) | (mg.) |
|---|---|---|
| Active ingredient | 200.0 | 100.0 |
| Lactose | 211.0 | 105.5 |
| Corn Starch | 12.0 | 6.0 |
| Water (per thousand tablets) | (120 ml.)* | (60 ml.)* |
| Corn Starch | 75.0 | 37.5 |
| Magnesium Stearate | 2.0 | 1.0 |
| | 500.0 | 250.0 |

*(The water evaporates during manufacture.)

*Manufacturing Procedure:*

Blend the active ingredient with the lactose until uniform. Blend the smaller quantity of corn starch with the water and add the resulting corn starch paste, then mix until a uniform wet mass is formed. Add the remaining corn starch to the resulting wet mass and mix until uniform granules are obtained. Screen the granules through a suitable milling machine, using a 3/4" stainless stell screen. Dry the milled granules in a suitable drying oven until the desired moisture content is obtained. Mill the dried granules through a suitable milling machine using 16 mesh stainless steel screen. Blend in the magnesium stearate and compress the resulting mixture into tablets of desired shape, thickness, hardness and disintegration.

**Claims for the Contracting States: BE CH DE FR GB IT NL SE**

1. The (R,R) optical isomer of labetalol, namely 5 - {(R) - 1 - hydroxy - 2 - [(R) - (1 - methyl - 3 - phenylpropyl)amino]ethyl}salicylamide, said (R,R) optical isomer being substantially free of the corresponding (R,S), (S,R) and (S,S) optical isomers; and the pharmaceutically acceptable acid addition salts thereof, especially its hydrochloride, sulfate, maleate, tartrate, citrate, acetate, phthalate, succinate, lactate, malate, cinnamate, hydrobromide and phosphate.

2. 5 - {(R) - 1 - Hydroxy - 2 - [(R) - (1 - methyl - 3 - phenylpropyl)amino]ethyl}salicylamide, said (R,R) optical isomer being substantially free of the corresponding (R,S), (S,R) and (S,S) optical isomers; and its hydrochloride.

3. A process for the preparation of the (R,R) optical isomer claimed in claim 1 and of its pharmaceutically acceptable acid addition salts, substantially free of the corresponding (R,S), (S,R) and (S,S) optical isomers, which comprises removing the protecting groups from an N,O-protected (R,R)

optical isomer or acid addition salt thereof, the term "N,O-protected" indicating that the basic nitrogen atom and the phenolic hydroxy group are protected, and isolating the resulting (R,R) optical isomer as the free base or as a pharmaceutically acceptable acid addition salt.

4. A process as claimed in claim 3 wherein the step of removing the protecting groups is performed by hydrogenolysis, preferably by means of hydrogen and palladium on carbon, of protecting groups selected from N- or O-benzyl, N- or O-benzyloxycarbonyl or N-trichloroethoxycarbonyl groups, preferably of N- and O-benzyl groups.

5. A process as claimed in claim 3 or claim 4 wherein the N,O-protected (R,R) optical isomer has been obtained by resolution of an N,O-protected (R,R)-(S,R) diastereoisomeric mixture, by physical methods, preferably by chromatography on silica gel.

6. A process as claimed in claim 5 wherein the N,O-protected (R,R)-(S,R) diastereoisomeric mixture has been obtained by reduction of an N,O-protected 5 - {N-(R) - 1 - methyl - 3 - phenylpropyl)-glycyl}salicylamide, preferably by a borohydride in an organic solvent, more preferably by sodium borohydride in a lower alkanol.

7. A process as claimed in claim 6 wherein the N,O-protected 5 - {N - [(R) - 1 - methyl - 3 - phenylpropyl]glycyl}salicylamide has been obtained by condensation of a 4-O-protected $\alpha$-bromo-3-carbamoylacetophenone with an (R)-N-protected-1-methyl-3-phenylpropylamine.

8. A process for the preparation of the (R,R) optical isomer claimed in claim 1 and of its pharmaceutically acceptable acid addition salts, substantially free of the corresponding (R,S), (S,R) and (S,S) optical isomers, which comprises condensing a 4-O-protected-$\alpha$-bromo-3-carbamoylacetophenone with an N-protected-1-methyl-3-phenylpropylamine, obtaining an N,O-protected 5 - {N - [(R) - 1 - methyl - 3 - phenylpropyl]glycyl}salicylamide either by using an (R)-N-protected-1-methyl-3-phenylpropylamine in the foregoing condensation or by resolving the resulting racemic N,O-protected 5 - [N - 1 - methyl - 3 - phenylpropyl)glycyl]salicylamide, reducing the N,O-protected 5 - {N - [(R) - 1 - methyl - 3 - phenylpropyl]glycyl}salicylamide to a mixture of an N,O-protected 5 - {(R) - 1 - hydroxy - 2 - [(R) - (1 - methyl - 3 - phenylpropyl)amino]ethyl}salicylamide and the corresponding (S,R) optical isomer, separating from this mixture the N,O-protected 5 - {(R) - 1 - hydroxy - 2 - [(R) - (1 - methyl - 3 - phenylpropyl)amino]ethyl}salicylamide, removing the protecting groups therefrom and isolating 5 - {(R) - 1 - hydroxy - 2 - [(R) - (1 - methyl - 3 - phenylpropyl)amino]ethyl}salicylamide as the free base or as a pharmaceutically acceptable acid addition salt thereof; preferably the process comprising condensing 4 - benzyloxy - $\alpha$ - bromo - 3 - carbamoylacetophenone with (R) - (+) - N - benzyl - 1 - methyl - 3 - phenylpropylamine to yield 2 - O - benzyl - 5 - {N - benzyl - N - [(R) - 1 - methyl - 3 - phenylpropyl]glycyl}salicylamide, reducing this compound to a mixture of 2 - O - benzyl - 5 - {(R) - 1 - hydroxy - 2 - [(R) - (1 - methyl - 3 - phenylpropyl)benzylamino]ethyl}salicylamide and the corresponding (S,R) optical isomer, separating from this mixture 2 - O - benzyl - 5 - {(R) - 1 - hydroxy - 2 - [(R) - (1 - methyl - 3 - phenylpropyl)benzylamino]ethyl}salicylamide, removing the protecting N- and O-benzyl groups therefrom by hydrogenolysis and isolating 5 - {(R) - 1 - hydroxy - 2 - [(R) - (1 - methyl - 3 - phenylpropyl)amino]ethyl}salicylamide as the free base or as a pharmaceutically acceptable acid addition salt thereof.

9. Pharmaceutical compositions containing as active ingredient 5 - {(R) - 1 - hydroxy - 2 - [(R) - (1 - methyl - 3 - phenylpropyl)amino]ethyl}salicylamide, said (R,R) optical isomer being substantially free of the corresponding (R,S), (S,R) and (S,S) optical isomers, or a pharmaceutically acceptable acid addition salt thereof, together with a pharmaceutical carrier or excipient.

10. Compositions as claimed in claim 9 in the form of dosage units, e.g., tablets, capsules, pills, suppositories or injectable preparations in ampoules, containing preferably from 2 to 500 mg. of active ingredient per dosage unit.

11. N,O-protected 5 - {(R) - 1 - hydroxy - 2 - [(R) - (1 - methyl - 3 - phenylpropyl)amino]ethyl}salicylamides, diastereoisomeric mixtures of this (R,R) optical isomer with its (S,R) diastereoisomer, and N,O-protected 5 - {N - [(R) - 1 - methyl - 3 - phenylpropyl]glycyl}salicylamides, wherein the basic nitrogen atom and the phenolic hydroxy group are protected by hydrogenolysable or readily hydrolysable groups; preferably the compounds 2 - O - benzyl - 5 - {(R) - 1 - hydroxy - 2 - [(R) - (1 - methyl - 3 - phenylpropyl)benzylamino]ethyl}salicylamide and 2 - O - benzyl - 5 - {N - benzyl - N - [(R) - 1 - methyl - 3 - phenylpropyl]glycyl}salicylamide.

**Revendications pour les Etats contractants: BE CH DE FR GB IT NL SE**

1. Isomère optique (R,R) du labétalol, c'est-à-dire 5 - {(R) - 1 - hydroxy - 2 - [(R) - (1 - méthyl - 3 - phénylpropyl)amino]éthyl}salicylamide, ledit isomère optique (R,R) étant sensiblement dépourvu des isomères optiques (R,S), (S,R) et (S,S) correspondants, et ses sels d'addition d'acides acceptables en pharmacie, en particulier son chlorhydrate, son sulfate, son maléate, son tartrate, son citrate, son acétate, son phtalate, son succinate, son lactate, son malate, son cinnamate, son bromhydrate et son phosphate.

2. 5 - {(R) - 1 - hydroxy - 2 - [(R) - (1 - méthyl - 3 - phénylpropyl)amino]éthyl}salicylamide, ledit isomère optique (R,R) étant sensiblement dépourvu des isomères optiques (R,S), (S,R) et (S,S) correspondants; et son chlorhydrate.

3. Procédé de préparation de l'isomère optique (R,R) selon la revendication 1 et de ses sels d'addition d'acide acceptables en pharmacie, sensiblement dépourvu des isomères optiques correspondants (R,S), (S,R) et (S,S), qui consiste à retirer les groupes protecteurs d'un isomère optique (R,R) N,O-protégé ou de son sel d'addition d'acide, le terme "N,O-protégé" indiquant que l'atome d'azote basique et le groupe hydroxyphénolique sont protégés, et à isoler l'isomère optique (R,R) résultant sous forme de base libre ou sous forme d'un sel d'addition d'acide acceptable en pharmacie.

4. Procédé selon la revendication 3 du type où l'étape de retirer les groupes protecteurs est accomplie par hydrogènolyse, de préférence au moyen d'hydrogène et de palladium sur charbon, des groupes protecteurs choisis parmi les groupes N- ou O-benzyle, N- ou O-benzyloxycarbonyle ou N-trichloroéthoxycarbonyle, de préférence des groupes N- et O-benzyle.

5. Procédé selon l'une quelconque des revendications 3 ou 4, du type où l'isomère optique (R,R) N,O-protégé a été obtenu par résolution d'un mélange de diastéréoisomères (R,R)-(S,R) N,O-protégés par des procédés physiques, de préférence par chromatographie sur gel de silice.

6. Procédé selon la revendication 5 du type où le mélange de diastéréoisomères (R,R)-(S,R) N,O-protégés a été obtenu par réduction d'un 5 - {N - [(R) - 1 - méthyl - 3 - phénylpropyl]-glycyl}salicylamide N,O-protégé, de préférence par un borohydrate dans un solvent organique, et mieux par un borohydrate de sodium dans un alcanol inférieur.

7. Procédé selon la revendication 6, où le 5 - {N - [(R) - 1 - methyl - 3 - phenylpropyl]glycyl}salicylamide N,O-protégé a été obtenu par condensation d'une $\alpha$ - bromo - 3 - carbamoylacétophénone 4-O-protégée avec une (R) - N - protégée - 1 - méthyl - 3 - phénylpropylamine.

8. Procédé de préparation de l'isomère optique (R,R) selon la revendication 1 et de ses sels d'addition d'acides acceptables en pharmacie, sensiblement dépourvu des isomères optiques (R,S), (S,R) et (S,S) correspondants, qui consiste à condenser une 4 - O - protégée - $\alpha$ - bromo - 3 - carbamoylacétophénone avec une N - protégée - 1 - méthyl - 3 - phénylpropylamine, à obtenir un 5 - {N - [(R) - 1 - méthyl - 3 - phénylpropyl]glycyl}salicylamide soit en utilisant une (R) - N - protégée - 1 - méthyl - 3 - phénylpropylamine dans la condensation ci-dessus ou en résolvant le 5 - {N - 1 - méthyl - 3 - phénylpropyl]glycyl}salicylamide racémique N,O-protégé résultant, en réduisant le 5 - {N - [(R) - 1 - méthyl - 3 - phénylpropyl]glycyl}salicylamide N,O-protégé en un mélange d'un 5 - {(R) - 1 - hydroxy - 2 - [(R) - (1 - méthyl - 3 - phénylpropyl)amino]éthyl}salicylamide N,O-protégé et l'isomère optique (S,R) correspondant, en séparant, de ce mélange, le 5 - {(R) - 1 - hydroxy - 2 - [(R) - (1 - méthyl - 3 - phénylpropyl)amino]éthyl}salicylamide N,O-protégé, en en retirant les groupes protecteurs et en isolant le 5 - {(R) - 1 - hydroxy - 2 - [(R) - (1 - méthyl - 3 - phénylpropyl)amino]éthyl}salicylamide sous forme de base libre ou de son sel d'addition d'acide acceptable en pharmacie; le procédé consistant de préférence à condenser de la 4 - benzyloxy - $\alpha$ - bromo - 3 - carbamoylacétophénone avec de la (R) - (+) - N - benzyl - 1 - méthyl - 3 - phénylpropylamine pour donner du 2 - O - benzyl - 5 - {N - benzyl - N - [(R) - 1 - méthyl - 3 - phénylpropyl]glycyl}salicylamide, à réduire ce composé en un mélange de 2 - O - benzyl - 5 - {(R) - 1 - hydroxy - 2 - [(R) - (1 - méthyl - 3 - phénylpropyl)benzylamino]éthyl}salicylamide et l'isomère optique (S,R) correspondant, à séparer dudit mélange, le 2 - O - benzyl - 5 - {(R) - 1 - hydroxy - 2 - [(R) - (1 - méthyl - 3 - phénylpropyl)benzylamino]éthyl}salicylamide, à en retirer les groupes protecteurs N- et O-benzyle par hydrogénolyse et á isoler le 5 - {(R) - 1 - hydroxy - 2 - [(R) - (1 - méthyl - 3 - phénylprupyl)amino]éthyl}salicylamide sous forme de base libre ou de son sel d'addition d'acide acceptable en pharmacie.

9. Compositions pharmaceutiques contenant comme ingrédient actif le 5 - {(R) - 1 - hydroxy - 2 - [(R) - (1 - méthyl - 3 - phénylpropyl)amino]éthyl}salicylamide, ledit isomère optique (R,R) étant sensiblement dépourvu des isomères optiques (R,S), (S,R) et (S,S) correspondants, ou son sel d'addition d'acide acceptable en pharmacie, avec un véhicule ou excipient pharmaceutique.

10. Compositions selon la revendication 9 sous forme de doses unitaires, telles que des comprimés, capsules, pilules, suppositoires ou préparations injectables en ampoules, contenant de préférence de 2 à 500 mg de l'ingrédient actif par dose unitaire.

11. 5 - {(R) - 1 - hydroxy - 2 - [(R) - (1 - méthyl - 3 - phényl-propyl)amino]éthyl}salicylamides N,O-protégés, mélanges de diastéréoisomères de cet isomère optique (R,R) avec son diastéréoisomère (S,R), et 5 - {(N - [(R) - 1 - méthyl - 3 - phényl-propyl)]glycyl}salicylamides N,O-protégés, du type où l'atome d'azote basique et le groupe hydroxyphénolique sont protégés par des groupes hydrogénolysables ou facilemênt hydrolysables, de préférence les composés 2 - O - benzyl - 5 - {(R)) - 1 - hydroxy - 2 - [(R) - (1 - méthyl - 3 - phénylpropyl)benzylamino]éthyl}salicylamide et 2 - O - benzyl 5 - {N - benzyl - N - [(R) - 1 - méthyl - 3 - phénylpropyl]glycyl}salicylamide.

**Patentansprüche für die Vertragsstaaten BE CH DE FR GB IT NL SE**

1. Das (R,R)-optische Isomere des Labetalols, nämlich 5 - {(R) - 1 - Hydroxy - 2 - [(R) - (1 - methyl - 3 - phenylpropyl)amino]äthyl}salicylamid, wobei das (R,R)-optische Isomere praktisch frei von den entsprechenden (R,S)-, (S,R)- und (S,S)-optischen Isomeren ist, sowie dessen pharmazeutisch unbedenkliche Säureadditionssalze, insbesondere sein Hydrochlorid, Sulfat, Maleat, Tartrat, Citrat, Acetat, Phthalat, Succinat, Lactat, Malat, Cinnamat, Hydrobromid und Phosphat.

2. 5 - {(R) - 1 - Hydroxy - 2 - [(R) - (1 - methyl - 3 - phenylpropyl)amino]äthyl}salicylamid, wobei das (R,R)-optische Isomere praktisch frei von den entsprechenden (R,S)-, (S,R)- und (S,S)-optischen Isomeren ist, sowie sein Hydrochlorid.

3. Verfahren zur Herstellung des in Anspruch 1 beanspruchten (R,R)-optischen Isomeren und seiner pharmazeutisch unbedenklichen Säureadditionssalze, praktisch frei von den entsprechenden (R,S)-, (S,R)- und (S,S)-optischen Isomeren, dadurch gekennzeichnet, daß die Schutzgruppen von einem N,O-geschützten (R,R)-optischen Isomeren oder dessen Säureadditionssalz, worin der Begriff "N,O-geschützt" bedeutet, daß das basische Stickstoff-Atom und die phenolische Hydroxyl-Gruppe geschützt sind, entfernt werden und das erhaltene (R,R)-optische Isomere als freie Base oder als ein pharmazeutisch unbedenkliches Säureadditionssalz isoliert wird.
end of tape two

4. Verfahren nach Anspruch 3, dadurch gekennzeichnet, daß die Entfernung der Schutzgruppen durch Hydrogenolyse, vorzugsweise mit Hilfe von Wasserstoff und Palladium auf Kohlenstoff, durchgeführt wird, wobei die Schutzgruppen aus N- oder O- Benzyl, N- oder O-Benzyloxycarbonyl- oder N-Trichloräthoxy carbonyl-Gruppen, vorzugsweise aus N- und O-Benzyl-Gruppen bestehen.

5. Verfahren nach Anspruch 3 oder 4, dadurch gekennzeichnet, daß das N,O-geschützte (R,R)-optische Isomere durch Auftrennung eines N,O-geschützten (R,R)- (S,R)-Diastereoisomeren-Gemischs mittels physikalischer Methoden, vorzugsweise durch Chromatographie an Silica-Gel erhalten wurde.

6. Verfahren nach Anspruch 5, dadurch gekennzeichnet, daß das N,O-geschützte (R,R)- (S,R)-Diastereoisomeren-Gemisch durch Reduktion eines N,O-geschützten 5 - {N - [(R) - 1 - Methyl - 3 - phenylpropyl]glycyl}salicylamids, vorzugsweise durch ein Borhydrid in einem organischen Lösungsmittel, besonders bevorzugt durch Natriumborhydrid in einem niederen Alkanol, erhalten wurde.

7. Verfahren nach Anspruch 6, dadurch gekennzeichnet, daß das N,O-geschützte 5 - {N - [(R) - 1 - Methyl - 3 - phenylpropyl]glycyl}salicylamid durch Kondensation eines 4 - O - geschützten $\alpha$ - Brom - 3 - carbamoylacetophenons mit einem (R) - N - geschützten 1 - Methyl - 3 - phenylpropylamin erhalten wurde.

8. Verfahren zur Herstellung des (R,R)-optischen Isomeren nach Anspruch 1 und seiner pharmazeutisch unbedenklichen Säureadditionssalze, praktisch frei von den entsprechenden (R,S)-, (S,R)- und (S,S)-optischen Isomeren, dadurch gekennzeichnet, daß man ein 4 - O - geschütztes $\alpha$ - Brom - 3 - carbamoylacetophenon mit einem N-geschützten 1 - Methyl - 3 - phenylpropylamin kondensiert, ein N,O-geschütztes 5 - {N - [(R) - 1 - Methyl - 3 - phenylpropyl]glycyl}salicylamid entweder durch Verwendung eines (R) - N - geschützten 1 - Methyl - 3 - phenylpropylamins in der vorhergehenden Kondensation oder durch Auftrennung des erhaltenen razemischen N,O-geschützten 5 - [N - (1 - Methyl - 3 - phenylpropyl)glycyl]salicylamids erhält, das N,O-geschützte 5 - {N - [(R) - 1 - Methyl - 3 - phenylpropyl]glycyl}salicylamid zu einer Mischung aus einem N,O-geschützten 5 - {(R) - 1 - Hydroxy - 2 - [(R) - (1 - methyl - 3 - phenylpropyl)amino]äthyl}salicylamid und den entsprechenden (S,R)-optischen Isomeren reduziert, aus diesem Gemisch das N,O-geschützte 5 - {(R) - 1 - Hydroxy - 2 - [(R) - (1 - methyl - 3 - phenylpropyl)amino]äthyl}salicylamid abtrennt, die Schutzgruppen aus diesem entfernt und 5 - {(R) - 1 - Hydroxy - 2 - [(R) - (1 - methyl - 3 - phenylpropyl)amino]äthyl}salicylamid als freie Base oder als eines ihrer pharmazeutisch unbedenklichen Säureadditionssalze isoliert, sowie vorzugsweise dadurch gekennzeichnet, daß man 4-Benzyloxy- $\alpha$-brom -3-carbamoyl-acetophenon mit (R) - (+) - N - Benzyl - 1 - methyl - 3 - phenyl - propylamin unter Bildung von 2 - O - Benzyl - 5 - {N - benzyl - N - [(R) - 1 - methyl - 3 - phenylpropyl]glycyl}salicylamid kondensiert, diese Verbindung zu einer Mischung aus 2 - O - Benzyl - 5 - {(R) - 1 - hydroxy - 2 - [(R) - (1 - methyl - 3 - phenylpropyl)benzylamino]äthyl}salicylamid und dem entsprechenden (S,R)-optischen Isomeren reduziert, aus diesem Gemisch das 2 - O - Benzyl - 5 - {(R) - 1 - hydroxy - 2 - [(R) - (1 - methyl - 3 - phenylpropyl)benzylamino]äthyl}salicylamid abtrennt, die N- und O-Benzyl-Schutzgruppen daraus durch Hydrolyse entfernt und 5 - {(R) - 1 - Hydroxy - 2 - [(R) - (1 - methyl - 3 - phenylpropyl)amino]äthyl}salicylamid als freie Base oder als deren pharmazeutisch unbedenkliches Säureadditionssalz isoliert.

9. Pharmazeutische Zubereitungen, enthaltend als wirksamen Inhaltsstoff 5 - {(R) - 1 - Hydroxy - 2 - [(R) - (1 - methyl - 3 - phenylpropyl)amino]äthyl}salicylamid, wobei das (R,R)-optische Isomere praktisch frei von den entsprechenden (R,S)-, (S,R)- und (S,S)-optischen Isomeren ist, oder eines seiner pharmazeutisch unbedenklichen Säureadditionssalze zusammen mit einem pharmazeutischen Träger oder Streckmittel.

10. Zubereitungen nach Anspruch 9 in Form von Dosierungseinheiten, z.B. Tabletten, Kapseln, Pillen, Suppositorien oder injizierbaren Präparaten in Ampullen, die vorzugsweise von 2—500 mg des wirksamen Inhaltsstoffes pro Dosierungseinheit enthalten.

11. N,O-geschützte 5 - {(R) - 1 - Hydroxy - 2 - [(R) - (1 - methyl - 3 - phenylpropyl)amino]äthyl}salicylamide, diastereoisomere Gemische dieses (R,R)-optischen Isomeren mit ihrem (S,R)-Diastereoisomeren und N,O-geschützte 5 - {N - [(R) - 1 - Methyl - 3 - phenylpropyl]glycyl}salicylamide, worin das basische Stickstoff-Atom und die phenolische Hydroxy-Gruppe durch hydrogenolysierbare oder leicht hydrolisierbare Gruppen geschützt sind, vorzugsweise die Verbindungen 2 -O - Benzyl - 5 - {(R) - 1 - hydroxy - 2 - [(R) - (1 - methyl - 3 - phenylpropyl)benzylamino]äthyl}salicylamid und 2 - O - Benzyl - 5 - {N - Benzyl - N - [(R) - 1 - methyl - 3 - phenylpropyl]glycyl}salicylamid.

**Claims for the Contracting State: AT**

1. A process for the preparation of the (R,R) optical isomer of labetalol, namely 5 - {(R) - 1 - hydroxy - 2 - [(R) - (1 - methyl - 3 - phenylpropyl)amino]ethyl}salicylamide, said (R,R) optical isomer being substantially free of the corresponding (R,S), (S,R) and (S,S) optical isomers, and of the pharmaceutically acceptable acid addition salts thereof, which comprises removing the protecting groups from an N,O-protected (R,R) optical isomer or acid addition salt thereof, the term "N,O-protected" indicating that the basic nitrogen atom and the phenolic hydroxy group are protected, and isolating the resulting (R,R) optical isomer as the free base or as a pharmaceutically acceptable acid addition salt.

2. A process as claimed in claim 1 wherein the step of removing the protecting groups is performed by hydrogenolysis, preferably by means of hydrogen and palladium on carbon, of protecting groups selected from N- or O-benzyl, N- or O-benzyloxycarbonyl or N-trichloroethoxycarbonyl groups, preferably of N- and O-benzyl groups.

3. A process as claimed in claim 1 or claim 2 wherein the N,O-protected (R,R) optical isomer has been obtained by resolution of an N,O-protected (R,R)-(S,R) diastereoisomeric mixture, by physical methods, preferably by chromatography on silica gel.

4. A process as claimed in claim 3 wherein the N,O-protected (R,R)-(S,R) diastereoisomeric mixture has been obtained by reduction of an N,O-protected 5 - {N - [(R) - 1 - methyl - 3 - phenylpropyl]glycyl}salicylamide, preferably by a borohydride in an organic solvent, more preferably by sodium borohydride in a lower alkanol.

5. A process as claimed in claim 4 wherein the N,O-protected 5 - {N - [(R) - 1 - methyl - 3 - phenylpropyl]glycyl}salicylamide has been obtained by condensation of a 4-O-protected $\alpha$-bromo-3-carbamoylacetophenone with an (R) - N - protected - 1 - methyl - 3 - phenylpropylamine.

6. A process for the preparation of the (R,R) optical isomer of labetalol and of its pharmaceutically acceptable acid addition salts, substantially free of the corresponding (R,S), (S,R) and (S,S) optical isomers, which comprises condensing a 4 - O - protected - $\alpha$ - bromo - 3 - carbamoylacetophenone with an N - protected - 1 - methyl -3 - phenylpropylamine, obtaining an N,O-protected 5 - {N - [(R) - 1 - methyl - 3 - phenylpropyl]glycyl}salicylamide either by using an (R) - N - protected - 1 - methyl - 3 - phenylpropylamine in the foregoing condensation or by resolving the resulting racemic N,O-protected 5 - [N - 1 - methyl - 3 - phenylpropyl]glycyl}salicylamide, reducing the N,O-protected 5 - {N - [(R) - 1 - methyl - 3 - phenylpropyl]glycyl}salicylamide to a mixture of an N,O-protected 5 - {(R) - 1 - hydroxy - 2 - [(R) - (1 - methyl - 3 - phenylpropyl)amino]ethyl}-salicylamide and the corresponding (S,R) optical isomer, separating from this mixture the N,O-protected 5 - {(R) - 1 - hydroxy - 2 - [(R) - 1 - methyl - 3 - phenylpropyl)amino]ethyl}salicylamide, removing the protecting groups therefrom and isolating 5 - {(R) - 1 - hydroxy - 2 - [(R) - (1 - methyl - 3 - phenylpropyl)amino]ethyl}salicylamide as the free base or as a pharmaceutically acceptable acid addition salt thereof; preferably the process comprising condensing 4 - benzyloxy - $\alpha$ - bromo - 3 - carbamoyl - acetophenone with (R) - (+) - N - benzyl - 1 - methyl - 3 - phenylpropylamine to yield 2 - O - benzyl - 5 - {N - benzyl - N - [(R) - 1 - methyl - 3 - phenylpropyl]-glycyl}salicylamide, reducing this compound to a mixture of 2 - O - benzyl - 5 - [(R) - 1 - hydroxy - 2 - [(R) - (1 - methyl - 3 - phenylpropyl)benzylamino]ethyl}salicylamide and the corresponding (S,R) optical isomer, separating from this mixture 2 - O - benzyl - 5 - {(R) - 1 - hydroxy - 2 - [(R) - (1 - methyl - 3 - phenylpropyl)benzylamino]ethyl}salicylamide, removing the protecting N- and O-benzyl groups therefrom by hydrogenolysis and isolating 5 - {(R) - 1 - hydroxy - 2 - [(R) - (1 - methyl - 3 - phenylpropyl)amino]ethyl}salicylamide as the free base or as a pharmaceutically acceptable acid addition salt thereof.

7. A process as claimed in any of claims 1 to 6 wherein the (R,R) optical isomer of labetalol is isolated in the form of its sulfate, maleate, tartrate, citrate, acetate, phthalate, succinate, lactate, malate, cinnamate, hydrobromide, phosphate, or especially in the form of its hydrochloride.

8. A process for the preparation of a pharmaceutical composition containing as active ingredient 5 - {(R) - 1 - hydroxy - 2 - [(R) - (1 - methyl - 3 - phenylpropyl)amino]ethyl}salicylamide, said (R,R) optical isomer being substantially free of the corresponding (R,S), (S,R) and (S,S) optical isomers, or a pharmaceutically acceptable acid addition salt thereof, characterised in that the said active ingredient is admixed with a pharmaceutical carrier or excipient.

9. A process as claimed in claim 8 wherein the composition is prepared in the form of dosage units, e.g., tablets, capsules, pills, suppositories or injectable preparations in ampoules, containing preferably from 2—500 mg. of active ingredient per dosage unit.

10. A process as claimed in claim 8 or claim 9 wherein the active ingredient has been prepared by a process as claimed in any of claims 1 to 7.

**Revendications pour l'Etat Contractant: AT**

1. Procédé de préparation de l'isomère optique (R,R) du labétalol, c'est-à-dire 5 - {(R) - 1 - hydroxy - 2 - [(R) - (1 - méthyl - 3 - phénylpropyl)amino]éthyl}salicylamide, ledit isomère optique (R,R) étant sensiblement dépourvu des isomères optiques (R,S), (S,R) et (S,S) correspondants, et de ses sels d'addition d'acides acceptables en pharmacie qui consiste à retirer les groupes protecteurs d'un isomère optique (R,R) N,O-protégé ou de son sel d'addition d'acide, le terme "N,O-protégé" indiquant que l'atome d'azote basique et le groupe hydroxy phénolique sont protégés, et à isoler l'isomère optique (R,R) résultant sous forme de base libre ou sous forme d'un sel d'addition d'acide acceptable en pharmacie.

2. Procédé selon la revendication 1 du type où l'étape de retirer les groupes protecteurs est accomplie par hydrogènolyse, de préférence au moyen d'hydrogène et de palladium sur charbon, des groupes protecteurs choisis parmi les groupes N- ou O-benzyle, N- ou O-benzyloxycarbonyle ou N-trichloroéthoxycarbonyle, de préférence des groupes N- et O-benzyle.

3. Procédé selon l'une quelconque des revendications 1 ou 2, du type où l'isomère optique (R,R) N,O-protégé a été obtenu par résolution d'un mélange de diastéréoisomères (R,R)-(S,R) N,O-protégés par des procédés physiques, de préférence par chromatographie sur gel de silice.

4. Procédé selon la revendication 3 du type où le mélange de diastéréoisomères (R,R)-(S,R) N,O-protégés a été obtenu par réduction d'un 5 - {N - [(R) - 1 - méthyl - 3 - phénylpropyl]glycyl}salicylamide N,O-protégé, de préférence par un borohydrate dans un solvant organique, et mieux par un borohydrate de sodium dans un alcanol inférieur.

5. Procédé selon la revendication 4, où le 5 - {N - [(R) - 1 - méthyl - 3 - phénylpropyl]glycyl}salicylamide N,O-protégé a été obtenu par condensation d'une $\alpha$ - bromo - 3 - carbamoylacétophénone 4-O-protégée avec une (R) - N - protégée - 1 - méthyl - 3 - phénylpropylamine.

6. Procédé de préparation de l'isomère optique (R,R) de labétalol et de ses sels d'addition d'acides acceptables en pharmacie, sensiblement dépourvu des isomères optiques (R,S), (S,R), et (S,S) correspondants, qui consiste à condenser une 4 - O - protégée - $\alpha$ - bromo - 3 - carbamoylacétophénone avec une N - protégée - 1 - méthyl - 3 - phénylpropylamine, à obtenir un 5 - {N - [(R) - 1 - méthyl - 3 - phénylpropyl]glycyl}salicylamide soit en utilisant une (R) - N - protégée - 1 - méthyl - 3 - phénylpropylamine dans la condensation ci-dessus ou en résolvant le 5 - [N - (1 - méthyl - 3 - phénylpropyl]glycyl}salicylamide racémique N,O-protégé résultant, en réduisant le 5 - {N - [(R) - 1 - méthyl - 3 - phénylpropyl]glycyl}salicylamide N,O-protégé en un mélange d'un 5 - {(R) - 1 - hydroxy - 2 - [(R) - (1 - méthyl - 3 - phénylpropyl)amino]éthyl}salicylamide N,O-protégé et l'isomère optique (S,R) correspondant, en séparant, de ce mélange le 5 - {(R) - 1 - hydroxy - 2 - [(R) - (1 - méthyl - 3 - phénylpropyl)amino]éthyl}salicylamide N,O-protégé, en en retirant les groupes protecteurs et en isolant le 5 - {(R) - 1 - hydroxy - 2 - [(R) - (1 - méthyl - 3 - phénylpropyl)amino]éthyl}salicylamide sous forme de base libre ou de son sel d'addition d'acide acceptable en pharmacie; le procédé consistant de préférence à condenser de la 4 - benzyloxy - $\alpha$ - bromo - carbamoylacétophénone avec de la (R) - (+) - N - benzyl - 1 - méthyl - 3 - phénylpropylamine pour donner du 2 - O - benzyl - 5 - {N - benzyl - N - [(R) - 1 - méthyl - 3 - phénylpropyl]glycyl}salicylamide, à réduire ce composé en un mélange de 2 - O - benzyl - 5 - {(R) - 1 - hydroxy - 2 - [(R) - (1 - méthyl - 3 - phénylpropyl)-benzylamino]éthyl}salicylamide et l'isomère optique (S,R) correspondant, à séparer dudit mélange, le 2 - O - benzyl - 5 - {(R) - 1 - hydroxy - 2 - [(R) - (1 - méthyl - 3 - phénylpropyl)benzylamino]éthyl}salicylamide, à en retirer les groupes protecteurs N- et O-benzyl par hydrogénolyse et à isoler le 5 - {(R) - 1 - hydroxy - 2 - [(R) - (1 - méthyl - 3 - phénylpropyl)amino]éthyl}salicylamide sous forme de base libre ou de son sel d'addition d'acide acceptable en pharmacie.

7. Procédé selon l'une quelconque des revendications 1 à 6 où l'isomère optique (R,R) de labétalol est isolé sous la forme de son sulfate, maléate, tartrate, citrate, acétate, phtalate, succinate, lactate, malate, cinnamate, bromhydrate, phosphate ou en particulier sous forme de son chlorhydrate.

8. Procédé de préparation d'une composition pharmaceutique contenant comme ingrédient actif le 5 - {(R) - 1 - hydroxy - 2 - [(R) - (1 - méthyl - 3 - phénylpropyl)amino]éthyl}salicylamide, ledit isomére optique (R,R) étant sensiblement dépourvu des isomères optiques (R,S), (S,R) et (S,S) correspondants ou un sel d'addition d'acide acceptable en pharmacie, caractérisé en ce que ledit ingrédient actif est mélangé avec un véhicule ou excipient pharmaceutique.

9. Procédé selon la revendication 8 préparé sous forme de doses unitaires, telles que des

comprimés, capsules, pilules, suppositoires ou préparations injectables en ampoules, contenant de préférence de 2 à 500 mg de l'ingrédient actif par dose unitaire.

10. Procédé selon l'une des revendications 8 ou 9 où l'ingrédient actif a été préparé par un procédé selon l'une quelconque des revendications 1 à 7.

**Patentansprüche für den Vertragsstaat: AT**

1. Verfahren zur Herstellung des (R,R)-optischen Isomeren des Labetalols, nämlich 5 - {(R) - 1 - Hydroxy - 2 - [(R) - (1 - methyl - 3 - phenylpropyl)amino]äthyl}salicylamid, wobei das (R,R)-optische Isomere praktisch frei von den entsprechenden (R,S)-, (S,R)- und (S,S)-optischen Isomeren ist und seiner pharmazeutisch unbedenklichen Säureadditionssalze, dadurch gekennzeichnet, daß die Schutzgruppen von einem N,O-geschützten (R,R)-optischen Isomeren oder dessen Säureadditionssalz, worin der Begriff "N,O-geschützt" bedeutet, daß das basische Stickstoff-Atom und die phenolische Hydroxylgruppe geschützt sind, entfernt werden und das erhaltene (R,R)-optische Iosmere als freie Base oder als ein pharmazeutisch unbedenkliches Säureadditionssalz isoliert wird.

2. Verfahren nach Anspruch 1, dadurch gekennzeichnet, daß die Entfernung der Schutzgruppen durch Hydrogenolyse, vorzugsweise mit Hilfe von Wasserstoff und Palladium auf Kohlenstoff, durchgeführt wird, wobei die Schutzgruppen aus N- oder O-Benzyl, N- oder O-Benzyloxycarbonyl- oder N-Trichloräthoxycarbonyl-Gruppen, vorzugsweise aus N- und O-Benzyl-Gruppen bestehen.

3. Verfahren nach Anspruch 1 oder 2, dadurch gekennzeichnet, daß das N,O-geschützte (R,R)-optische Isomere durch Auftrennung eines N,O-geschützten (R,R)- (S,R)-Diastereoisomeren-Gemischs mittels physikalischer Methoden, vorzugsweise durch Chromatographie an Silica-Gel, erhalten wurde.

4. Verfahren nach Anspruch 3, dadurch gekennzeichnet, daß das N,O-geschützte (R,R)-Diastereoisomeren-Gemisch durch Reduktion eines N,O-geschützten 5 - {N - [(R) - 1 - Methyl - 3 - phenylpropyl]glycyl}salicylamids, vorzugsweise durch ein Borhydrid in einem organischen Lösungsmittel, besonders bevorzugt durch Natriumborhydrid in einem niederen Alkanol erhalten wurde.

5. Verfahren nach Anspruch 4, dadurch gekennzeichnet, daß das N,O-geschützte 5 - {N - [(R) - 1 - Methyl - 3 - phenylpropyl]glycyl}salicylamid durch Kondensation eines 4 - O - geschützten $\alpha$ - Brom - 3 - carbamoylacetophenons mit einem (R)-N-geschützten 1 - Methyl - 3 - phenylpropylamin erhalten wurde.

6. Verfahren zur Herstellung des (R,R)-optischen Isomeren des Labetalols und seiner pharmazeutisch unbedenklichen Säureadditionssalze, praktisch frei von den entsprechenden (R,S)-, (S,R)-optischen Isomeren, dadurch gekennzeichnet, daß man ein 4-O-geschütztes $\alpha$ - Brom - carbamoylacetophenon mit einem N-geschützten 1 - Methyl - 3 - phenylpropylamin kondensiert, ein N,O-geschütztes 5 - {N - [(R) - 1 - Methyl - 3 - phenylpropyl]glycyl}salicylamid entweder durch Verwendung eines (R)-N-geschützten 1 - Methyl - 3 - phenylpropylamins in der vorhergehenden Kondensation oder durch Auftrennung des erhaltenen razemischen N,O-geschützten 5 - {N - (1 - Methyl - 3 - phenylpropyl]glycyl}salicylamids erhält, das N,O-geschützte 5 - {N - [(R) - 1 - Methyl - 3 - phenylpropyl]glycyl}salicylamid zu einer Mischung aus einem N,O-geschützten 5 - {(R) - 1 - Hydroxy - 2 - [(R) - (1 - methyl - 3 - phenylpropyl)amino]äthyl}salicylamid und den entsprechenden (S,R)-optischen Isomeren reduziert, aus diesem Gemisch das N,O-geschützte 5 - {(R) - 1 - Hydroxy - 2 - [(R) - (1 - methyl - 3 - phenylpropyl)amino]äthyl}salicylamid abtrennt, die Schutzgruppen aus diesem entfernt und 5 - {(R) - 1 - Hydroxy - 2 - [(R) - (1 - methyl - 3 - phenylpropyl)amino]äthyl}salicylamid als freie Base oder als eines ihrer pharmazeutisch unbedenklichen Säureadditionssalze isoliert, sowie vorzugsweise dadurch gekennzeichnet, daß man 4 - Benzyloxy - $\alpha$ - brom - 3 - carbamoylacetophenon mit (R) - (+) - N - Benzyl - 1 - methyl - 3 - phenyl - propylamin unter Bildung von 2 - O - Benzyl - 5 - {N - [(R) - 1 - methyl - 3 - phenylpropyl]glycyl}salicylamid kondensiert, diese Verbindung zu einer Mischung aus 2 - O - Benzyl - 5 - {(R) - 1 - hydroxy - 2 - [(R) - (1 - methyl - 3 - phenylpropyl)benzylamino]äthyl}salicylamid und dem entsprechenden (S,R)-optischen Isomeren reduziert, aus diesem Gemisch das 2 - O - Benzyl - 5 - {(R) - 1 - hydroxy - 2 - [(R) - (1 - methyl - 3 - phenylpropyl)benzylamino]äthyl}salicylamid abtrennt, die N- und O-Benzyl-Schutzgruppen daraus durch Hydrolyse entfernt und 5 - {(R) - 1 - Hydroxy - 2 - [(R) - (1 - methyl - 3 - phenylpropyl)amino]äthyl}salicylamid als freie Base oder als deren pharmazeutisch unbedenkliches Säureadditionssalz isoliert.

7. Verfahren nach Anspruch 1—6, dadurch gekennzeichnet, daß das (R,R)-optische Isomere des Labetalols in Form seines Sulfats, Maleats, Tartrats, Citrats, Acetats, Phthalats, Succinats, Lactats, Malats, Cinnamats, Hydrobromids, Phosphats oder insbesondere in Form seines Hydrochlorids isoliert wird.

8. Verfahren zur Herstellung einer pharmazeutischen Zubereitung, die als wirksamen Inhaltsstoff 5 - {(R) - 1 - Hydroxy - 2 - [(R) - (1 - methyl - 3 - phenylpropyl)amino]äthyl}salicylamid, wobei das (R,R)-optische Isomere praktisch frei von den entsprechenden (R,S), (S,R) und (S,S)-optischen Isomeren ist, oder eines seiner pharmazeutisch unbedenklichen Säureadditionssalze enthält, dadurch gekennzeichnet, daß der wirksame Inhaltsstoff mit einem pharmazeutischen Träger oder Streckmittel gemischt wird.

9. Verfahren nach Anspruch 8, dadurch gekennzeichnet, daß die Zubereitung in Form von Dosierungseinheiten, z.B. Tabletten, Kapseln, Pillen, Suppositorien oder injizierbaren Präparaten in Ampullen hergestellt wird, die vorzugsweise von 2—500 mg des wirksamen Inhaltsstoffes pro Dosierungseinheit enthalten.

10. Verfahren nach Anspruch 8 oder 9, dadurch gekennzeichnet, daß der wirksame Inhaltsstoff mit Hilfe eines Verfahrens nach Anspruch 1—7 hergestellt wurde.